# EUROPEAN PATENT APPLICATION

(11) **EP 1 198 997 A2**
(43) Date of publication of application: **24.04.2002**
(21) Application number: 01202990.6
(22) Date of filing: 07.08.2001
(51) Int. Cl.: A23L 3/32

(54) **Pulse sterilisation apparatus**

(30) Priority: 08.08.2000 NL 1015898
(71) Applicant: IV-Consult B.V., 3350 CD Papendrecht (NL); N.V. Kema, 6812 AR Arnhem (NL)
(72) Inventor: Van Houten, Hubertus, 3353 BM Papendrecht (NL)
(74) Representative: Jorritsma, Ruurd

(57) **Abstract**

Pulse sterilisation apparatus (1) for sterilising a fluid product comprising a housing provided with an inlet piece (19) and an outlet piece (9), an inner electrode (2) positioned in between the inlet piece (19) and the outlet piece (9) and an outer electrode (31) positioned around the inner electrode (2), in which the space between the inner electrode (2) and outer electrode (31) forms a treatment chamber (21) for the fluid product. The outer electrode (31) comprises a central bore of which the axis is parallel to a longitudinal direction of the pulse sterilisation apparatus (1) and in which the inner electrode (2) may be positioned, in which the central bore has a conical shape and the inner electrode (2) comprises a middle section (24) which has a conical shape at least in the region of the outer electrode (31), and in which the inner electrode (2) is adjustable in relation to the outer electrode (31).

## Description

The present invention relates to a pulse sterilisation apparatus for sterilising a fluid product comprising a housing provided with an inlet piece and an outlet piece, an inner electrode positioned in between the inlet piece and the outlet piece and an outer electrode positioned around the inner electrode, in which the space between the inner electrode and outer electrode forms a treatment chamber for the fluid product. The fluid product may be a liquid such as water or milk, but also a product having a higher viscosity such as a gel or a suspension of a fluid and other products such as manure.

Such a pulse sterilisation apparatus is known from American patent US-A-5.235.905. This patent describes an apparatus and method for elongating the storage life of pumpable food products. This is accomplished by exposing the pumpable food products to a pulsed high electric field. This high electric field results in damage to the cell walls of micro-organisms present in the food products, thereby sterilising the food products. The apparatus comprises two coaxial aligned electrodes, through which a cylinder shape treatment channel is formed. The outer electrode is grounded and also forms the housing of the apparatus. Furthermore, the treatment chamber comprises an inner electrode to which a high voltage generator may be connected. A middle section of the inner electrode forms in association with the outer electrode the treatment chamber, in which the distance between the outer electrode and the middle section of the inner electrode is substantially equal. The treatment chamber may be formed coaxial or tapered in the direction of the outflow opening. The apparatus is driven by a high voltage pulse generator, of which the pulse time and pulse frequency are controlled by a pulse generator, e.g. a micro computer. The food products are pumped through the apparatus such that all parts of the food products are exposed to at least one high voltage pulse. According to this American patent, an electrical field is generated in the food products of more than 25 kV/cm and preferably between 35 and 120 kV/cm. The distance between the two electrodes is at least 0.1 cm and is preferably between 0.5 and 3 cm. The pulse width of the high voltage pulse is between 0.1 and 25 µsec.

A disadvantage of this known apparatus is that because of the configuration chosen, the distance between the electrodes is fixed. The apparatus is thus not adaptable to the product to be treated.

The objective of the present application is thus to provide a pulse sterilisation apparatus in which the mentioned problems are not occurring.

This objective is achieved by a pulse sterilisation apparatus of the type as defined in the preamble, in which the outer electrode comprises a central bore of which the axis is parallel to a longitudinal direction of the pulse sterilisation apparatus and in which the inner electrode may be positioned, in which the central bore has a conical shape and the inner electrode comprises a middle section which has a conical shape at least in the region of the outer electrode, and in which the inner electrode is adjustable in relation to the outer electrode.

An advantage of the pulse sterilisation apparatus according to the present invention is that the distance between the inner electrode and the outer electrode is adjustable dependant on the product to be sterilised by shifting the inner and outer electrode with respect to each other in the longitudinal direction. Preferably, the conical shape of the central bore is equal to the conical shape of the middle section of the inner electrode, through which the distance between the inner electrode and outer electrode is substantially constant in all of the treatment chamber, and as a result of which a uniform electrical field is generated in the product to be sterilised.

In a preferred embodiment the conical shape of the central bore and the conical shape of the middle section of the inner electrode taper from the side of the outlet piece to the side of the inlet piece. As a result, a widening flow path for the product to be treated is formed in the flow direction, as a result of which a good flow is achieved without generation of vortex flows. Because of this, a more equal? Treatment of the product to be sterilised is possible.

In a further embodiment, the outer electrode is formed by a combination of at least one electrode plate which is surrounded on both sides by isolating members for electrically isolating the outer electrode from the housing. By applying multiple electrode plates, a better treatment of the product to be sterilised is possible, as it is possible to expose the product to high voltage pulses on multiple positions in the pulse sterilisation apparatus.

Preferably, the housing and the inner electrode are connected electrically with at least one connection for grounding the housing and the inner electrode. This results in a pulse sterilisation apparatus, which is safe during operation.

In a further embodiment of the pulse sterilisation apparatus according to the present invention, the central bore of the outer electrode and the inner electrode are provided with a layer dielectric material, such as ceramic material or plastic material, such as poly-ethylene or poly-propylene. As a result, possible occurring redox reactions in the product to be treated are prevented, and heat dissipation in the product to be treated in limited.

The pulse sterilisation apparatus according to the present invention further comprises at least one high voltage supply for providing high voltage pulses to the at least one electrode plate of the outer electrode. When the outer electrode comprises a combination of multiple electrode plates and isolating members, preferably a high voltage supply is used for each electrode plate. By using multiple electrode plates and separate high voltage supplies, varying voltages and varying pulse frequencies may be supplied to each electrode plate. This allows using the present apparatus to kill different micro organisms (fungi, yeast, bacteria and one cell forms such as amoebae and Naegleria fowleri) in a single treatment of the fluid product. The at least one high voltage supply is preferably driven by a pulse element, e.g. in the form of a computer or an industrial PLC control.

As high voltage supply, various solutions may be used, such as a combination of a circuit amplifier and a high voltage transformer (e.g. an ignition coil) connected to the circuit amplifier. As an alternative, a thyristor circuit may be used as high voltage supply. Both alternatives provide a reliable and economically producible pulse sterilisation apparatus.

Preferably, the high voltage supply provides high voltage pulses with a pulse frequency of 50-1500 pulses/sec. The pulse rate is adapted to the product to be treated. Preferably, the high voltage supply provides a voltage such that an electrical field is created in the product to be treated of between 10 and 50 kV/cm.

The pulse sterilisation apparatus according to the present invention will now be explained in more detail using a preferred embodiment, with reference to the accompanying drawing, in which:
Fig. 1 shows a sectional view of a pulse sterilisation apparatus according to the present invention;
Fig. 2 shows a diagrammatic view of an arrangement including the pulse sterilisation apparatus according to the invention in operation.

The function of the pulse sterilisation apparatus according to the present invention is killing micro-organisms, which are ubiquitarily present in high or low viscous fluids. A further advantage is that organoleptic properties and vitamins are not (or almost not) affected.

Bacterial cell walls are built up of polymers, such as the peptide glycan or murein complex. This macromolecule is a heteropolymer and comprises chains of N-Acetylglucosamine (GlcNAc) and a lactic acid ether of N-Acetylglucosamine, the N-Acetylmuramine acid (MurNAc). These are both coupled with each other β-1,4-glycosidic. These heteropolymer chains form the skeleton of the murein complex. The muramine acid compounds are connected peptic with the lactyl group to amino acid.

The group of these amino acids comprises L-alanine, D-glutamine acid, mesodiamine opimeline acid (Dpm) or L-lysine and D-alanine. The diamino acids m-, or LL-diaminopimeline acids and LL-lysine play an important role in the intermolecular connections as they from peptic connections with their amino groups and interconnect two heteropolymer chains in that way.

Although the invention is not limited to a specific explanation, it is assumed that exposure to the electrical pulse will amongst others lead to hydrolysis of the oxygen connection between N-acetylglucosamine (GlcNAc) and the N-acetylmuramine acid (MurNAc). This way, holes will be formed in the cell wall skeleton (murein complex), through which the cell contents may escape through the cell wall. Administering the electrical pulse can (further) break other inter or intra molecular connections form or between the various parts of the bacterial cell wall or at least disturb the structure of the cell wall such that this will become permeable for the cell contents.

Besides the magnitude of the voltage, also the length of the electrical pulses and the interval between the pulses is highly important. By means of an incorrect setting of the voltage, pulse length, pulse interval or a combination thereof, product damage in the form of vitamin destruction, protein denaturation and/or other severe damage to the organoleptic characteristics of the treated product will arise. Preferably, these parameters are chosen such that substantially none or almost no harm is done to the desired characteristics of the product to be sterilised, such as - in the case of food products - the taste, smell, consistency, structure, quality, etc.

The apparatus according to the present invention may be used to remove bacteria and/or other micro organisms form all desired fluids and fluid media, among which organic and/or aqueous fluids and media, as well as emulsions, suspensions, colloids and other mixtures capable of flowing. Some non-limiting examples are drinking water and waste water, fluid food products such as milk and other dairy products, drinks, soups, sauces, fluid food, baby and infant food etc; as well as medical and/or biological fluids and media, e.g. medicinal preparations, cosmetics, infusion fluids or growth media.

Here, the apparatus according to the invention may be used to remove all micro organisms or bacteria to be removed, amongst which gram positive or gram negative bacteria, but also e.g. fungi and spores thereof, and this will be clear for the person skilled in the art.

The invention is particularly suited for removing harmful micro-organisms from food products, such as food decaying bacteria or bacteria which harm or limit the quality or freshness of food products, process water and/or cooling water in another manner. Some non-limiting examples of such micro-organisms are Salmonella, Lysteria, Pseudomonas, Clostridium, S. Aureus, E.Coli.

In all of these applications, the apparatus according to the invention may be used analogous to, instead of or in addition to usual sterilisation systems or treatments, such as heat treatment or sterile filtration. The apparatus according to the invention can be used specifically for applications for which usual treatments are less suited, e.g. as the product to be sterilised is not well capable of undergoing high temperatures (such as food products) an/or is not well capable of being filtered (such as emulsions, suspensions, or high viscous preparations).

Examples of application fields in which the pulse sterilisation apparatus according to the present invention may be used will be apparent to the person skilled in the art, and comprise amongst others the food industry, dairy industry, drinks industry, drinking water production and control, sterilisation of manure, purification of wate water, process water and cooling water, and medical applications.

In waste water treatment the apparatus may be applied in effluent treatment to break a possibly present contamination cycle. Examples are prevention of amongst others contamination by Salmonella, amoebae and Naegleria fowleri. In drinking water preparation, e.g. as after treatment, applications in the third world or in ship installations, the apparatus may be used to prepare safe drinking water without E.coli, Salmonella, Cryptosporidium parvum and Legionella pneumophila. In water attractions, saunas, pools, public showers, the water may be protected against contamination with e.g. Legionella pneumophila. In the food product industry, sauces, soups, pulps, and cooling water may be conserved, and afterward contamination and/or cross contamination may be prevented. This specifically relates to protections against Mucor, Rhizopus nigrificans, Candida, Saccharomyces, Pseudomonas, etc. In the dairy industry, the apparatus may be applied for reducing the number of thermal treatments for killing harmful micro-organisms such as Pseudomonas, E.coli, Lactobacillaceae and Streptococcaceae. In the drinking industry, the apparatus may be applied for conserving drinks and fruit juices, providing protection against Lactobacillaceae, Candida en Saccharomyces. In the meat processing industry, afterward contamination and/or cross contamination by Enterobacteriaceae, Lactobacillaceae and Streptococcaceae in the rinsing water and/or flotation silt may be prevented. In manure processing the apparatus may be applied for reducing smell and for decontaminating and/or sterilising manure by Lactobacillaceae, Streptococcaceae and Clostridium.

Application of the invention generally causes a reduction in the quantity of viable micro-organisms in the medium with at least 50%, preferably at least 80% and with more preference at least 95% up to at least 99% or more. A possible measure for this is the so called germination number (i.e. for the one or more micro-organisms to be removed), which according to the invention is lowered with at least a factor 10, preferably with at least a factor 100 and more preferably with at least a factor 1000, e.g. depending on the number of viable micro-organisms which were originally present in the medium to be sterilised. In general, after treatment with the apparatus according to the invention, the germination number for the micro-organism to be removed will be reduced to a value of less than 100, preferably less than 50 and more preferably less than 10. For this, in general the (preferred) parameters as mentioned herein will be applied.

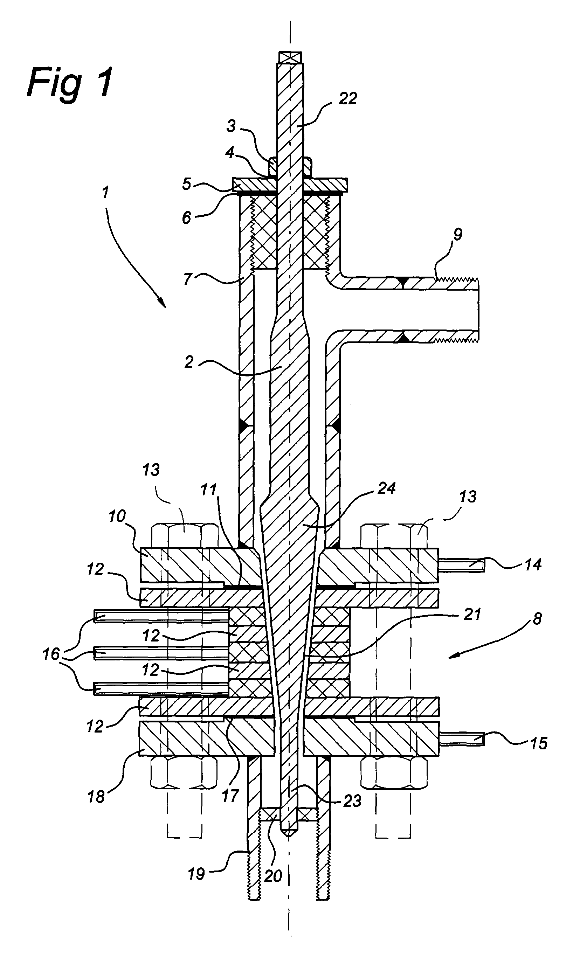

Fig. 1 shows a cross sectional view of a pulse sterilisation apparatus 1 according to the present invention. The pulse sterilisation apparatus 1 comprises an inlet piece 19 for the entrance of the fluid or suspension to be sterilised. Furthermore, the pulse sterilisation apparatus 1 comprises an outlet piece 9 for draining the fluid or suspension after treatment. Preferably, the outlet piece 9 is constructed as part of a pipe 7 formed as a T-piece, and the outlet piece 9 is perpendicular to the longitudinal direction of the sterilisation apparatus 1.

In the longitudinal direction of the pulse sterilisation apparatus 1, an arbour 2 is positioned which is preferably produced from a high quality rust free material, such as SS 316L. On the inner side of the inlet piece 19 a flow guide 20 is positioned, which centres the arbour 2 within the inlet piece 19. For this, the inlet piece 19 may be provided with a screw thread on the inside, and the flow guide 20 with a screw thread co-operating with that screw thread.

On the other side of the pulse sterilisation apparatus 1, the arbour 2 is centred in the ongoing part of T-piece 7 by a screw swivel 5 which is provided with an inner screw thread. The arbour 2 is provided with a screw thread an a first end 22, by which in co-operation with the inner screw thread of the screw swivel 5, the arbour 2 is adjustable in the longitudinal direction of the pulse sterilisation apparatus 1. The screw swivel 5 is mounted to the ongoing part of T piece 7, in which a seal 6 provides for a fluid tight sealing between the T-piece 7 and the screw swivel 5. A nut 3 provides for locking of the position of the arbour 2 in co-operation with a closing ring 4.

Between the inlet piece 19 and the T-piece 7, a middle section 8 is positioned with a top flange 10 which is connected to the T-piece 7 and a bottom flange 18 which is connected to the inlet piece 19. The connection may e.g. be a welding connection. Between the top flange 10 and bottom flange 18, a stack of electrodes 16 and isolating members 12 are positioned, an isolating member 12 being provided on both sides of the electrode 16 in the longitudinal direction of the pulse sterilisation apparatus 1. The stack of electrodes 16 and isolating members 12 is mounted between the top flange 10 and bottom flange 18 by means of a number of nuts and bolts 13. In the embodiment shown, the stack comprises three electrodes 16 and five isolating members 12. For the person skilled in the art, it will be clear that more or less electrodes 16 may be applied. Also, the multiple isolating members 12 may be formed in one piece, e.g. by injection moulding isolating material. The single isolating member then comprises openings which allow the electrodes 16 to be positioned such that the central bore is formed. Preferably, both for the top flange 10 and bottom flange 18 gaskets 11, 17 are used for sealing the stack.

Preferably, the top flange 10 is provided with a connection 14 for grounding the top flange 10, the T-piece 7, the outlet piece 9 as well as the arbour 2 via the screw swivel 5. The bottom flange 18 is preferably provided with a connection 15 which serves to ground the bottom flange 18 and the inlet piece 19. When bolts and nuts 13 are produced from the proper conducting material, possibly the connection 15 on the bottom flange 18 may be suspended with.

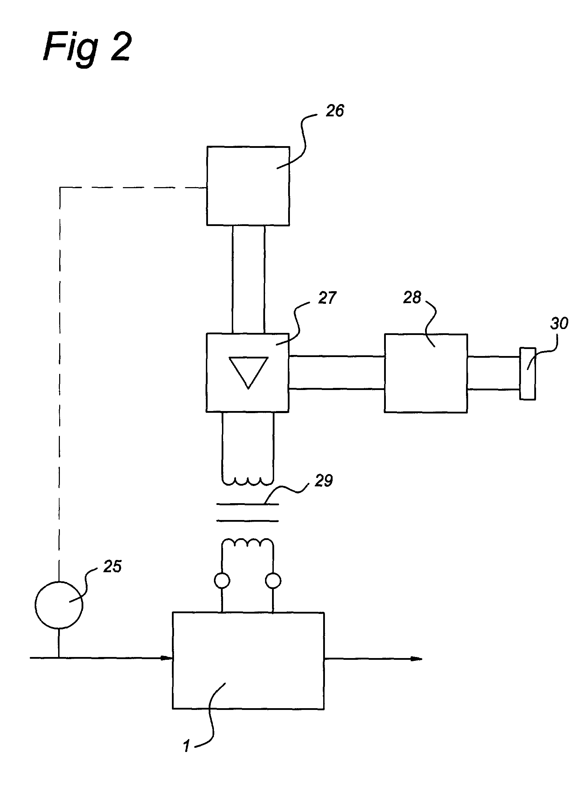

The electrodes 16 are provided with connections for connecting a high voltage supply 27, 29 (see discussion of Fig. 2 below). By the chosen arrangement of the electrodes 16 between the top and bottom flanges 10, 18, which are grounded, a safe operation of the pulse sterilisation apparatus 1 is possible. The arbour 2, is also grounded, via the housing of the pulse sterilisation apparatus 1 and no special measures have to be taken to obtain high voltage on the arbour 2.

The stack electrodes 16 and isolating members 12 has a central bore, in which the arbour 2 is positioned. The central bore preferably has a conical shape, in which the opening in the stack tapers from the top flange 10 towards the bottom flange 18. The arbour 2 has a middle section 24 with a diameter which tapers in the direction of the inlet piece 19. The angle between the surface of the middle part 24 of the arbour 2 and the axis of the pulse sterilisation apparatus 1 is preferably equal to the angle which the central bore of the stack forms with the axis, as a result of which the distance between the inner side of the stack electrodes 16 and isolating members 12 and the surface of the middle part 24 of the arbour 2 is substantially constant and forms a uniform treatment chamber 21. By adjusting the arbour 2 using the screw swivel 5 in the longitudinal direction, it is possible to change the distance between the surface of the middle part 24 of the arbour 2 and the stack electrodes 16 and isolating members 12. Preferably, the pitch of the inner screw thread of the screw swivel 5 (and thus the pitch of the screw thread of the end 22 of the arbour 2) and the conical shape of the middle part 24 of the arbour 2 is selected such that ten turns of the screw thread correspond to a change of the distance between the surface of the middle part 24 of the arbour 2 and the stack electrodes 16 and isolating members 12 of 1 mm.

For certain applications, the surface of the arbour 2 and/or the central bore of the stack of electrodes 16 and isolating members 12 may be covered by a dielectric layer of e.g. glass or Teflon, to prevent possible reactions with the fluid to be treated and to limit heat dissipation.

On each electrode 16, a connection is present for connecting a high voltage supply. The arbour 2 and the housing of the pulse sterilisation apparatus 1 (comprising inlet piece 19, bottom flange 18, top flange 10, T-piece 7 en outlet piece 9) are grounded by means of the connections 14, 15 on the top flange 10 and bottom flange 18.

The distance between the middle part 24 of the arbour 2 and the stack electrodes 16 and isolating members 12 in the treatment chamber 21 is continuously adjustable in dependence of the product to be treated. This has the additional advantage that during operation the pulse sterilisation apparatus 1 can be adapted to a changing conductivity of the product to be treated. Preferably, the pulse sterilisation apparatus 1 is arranged such that this distance is adjustable between 1.5 en 3.5 mm.

In an embodiment of the pulse sterilisation apparatus 1, 3 electrodes 16 and five isolating members 12 are present, which each have a thickness of 6 mm. The central bore of the stack electrodes 16 and isolating members 12 changes from a diameter of 10 mm at the side of bottom flange 18 to a diameter of 18.4 mm at the side of the top flange 10. Preferably the bore in the top flange also has a conical shape and changes from a diameter of 18.4 mm to a diameter of 20.94 mm. The arbour 2 preferably has an input section 23 with a diameter of 5 mm, which fits exactly in the flow guide 20. Furthermore, the arbour 2 comprises a tapered middle part 24, which changes from 5 mm diameter to 19.3 mm diameter. By the positioning of the arbour 2 in the pulse sterilisation apparatus 1, then a cylinder shaped treatment chamber 21 is formed at the height of the middle part 24 of the arbour 2, in which the distance between the surface of the middle part 24 of the (grounded) arbour 2 and the inner surface of the stack electrodes 16 and isolating members 12 is adjustable, e.g. between 1.5 and 3.5 mm.

The flow through opening of the pulse sterilisation apparatus 1 is limited in first instance in the embodiment shown by the difference in diameter of the inlet part of arbour 2 and the diameter of the bottom flange 18. In the embodiment shown, the diameter of the inlet part of arbour 2 is 5 mm and the diameter of the opening in the bottom flange 18 is 10 mm, which results in a flow through opening for the product of π(10/2)²-π(5/2)² = 58.9 mm². In second instance, the flow through of the pulse sterilisation apparatus 1 can be limited further by adjusting the arbour 2 inwardly, such that the distance between the middle part 24 of the arbour 2 and the inner surface of the stack electrodes 16 and isolating members 12 becomes smaller than 2.5 mm.

Fig. 2 shows a diagrammatic view of an arrangement with the pulse sterilisation apparatus 1 in operation. A supply 28 converts the line voltage of an AC voltage source 30 into a low DC voltage between 0 and 30 V, e.g. 12 V. Of course, other supply sources may be used, such as a battery. Preferably, the supply source 28 used is a supply source with high reliability.

Furthermore, a pulse generator 16 is present which provides DC voltage pulses with a frequency between 50 and 1500 or more pulses per second. The pulse generator 26 may be formed by a computer having an output, which provides the necessary pulses, or an industrial control, such as a PLC control. The pulse generator 26 should be able to generate all desired pulse times and intervals between the pulses (or pulse frequency and pulse width). In the embodiment shown, the pulse generator 26 is connected to a flow through meter 25 which measures the flow of fluid entering the pulse sterilisation apparatus 1. This allows adapting the characteristics of the pulses provided by the pulse generator 26 to the inflow of the fluid. Of course, other process parameters which may be measured using further measurement instruments 25 in front of, in or after the pulse sterilisation apparatus 1 may be used by the pulse generator 26 to adapt the characteristics of the pulses generated. In a further, alternative embodiment, the pulse generator 26 (e.g. in the form of a PLC control) may be arranged to adjust the arbour 2 of the pulse sterilisation apparatus 1 in the longitudinal direction, by means of which the flow through opening of the pulse sterilisation apparatus 1 may be controlled automatically.

Furthermore, a circuit amplifier 27 is present, preferably for each electrode 16, which is connected to the pulse generator 26 and to the supply source 28, and a high voltage transformer 29 which is connected to the circuit amplifier 27 and with to connections of the electrode 16. The circuit amplifier 27 is the coupling between the pulse generator 26, supply source 28 and the high voltage transformer 29. The circuit amplifier 27 may be a commercially available circuit amplifier, which is applied in the automotive industry. Such circuit amplifiers 27 are very reliable and readily available. The high voltage transformers 29 generate the necessary high voltage of 10...50 kV/cm required for operation of the pulse sterilisation apparatus 1. The high voltage transformers 29 may be generally available ignition coils, which are applied in the automotive industry, which are relatively cheap.

As an alternative, the pulses provided by the pulse generator 26 are converted to high voltage pulses for the electrodes 16 using solid state circuits, such as thyristors. These circuits then replace the circuit amplifiers 27 and the high voltage transformers in Fig. 2.

For evaluating the effectiveness of the pulse sterilisation apparatus according to the present invention, a number of tests have been executed. The tests relate to various products (water, raw milk, jelly (pectins) and faeces) with varying quantities and types of micro-organisms. In all tests the following parameters for the high voltage pulses were used: Electric field 42 kV/cm; pulse frequency 1500 Hz; and pulse length 2x10⁻⁶ sec.

In the following table, a list is given of the various tests executed, including a list of various micro-organisms and the reduction achieved of this number of micro-organisms after a single treatment with the pulse sterilisation apparatus 1 according to the present invention.

**Table I**

| **Overview tests and results** | |
|---|---|
| **Micro-organism** | **Reduction** |
| Streptococcaceae | 10⁹ |
| Salmonella | 10¹⁰ |
| Saccharomyces | 10⁸ |
| Pseudomonas alcaligenes | 10⁹ |
| Legionella pneumophila | 10¹⁰ |
| Lactobacillaceae | 10⁹ |
| Escherichia coli | 10¹⁰ |
| Enterobacteriaceae | 10¹⁰ |
| Enterobacter aerogens | 10⁸ |
| Other single celled organisms | 10¹² |
| Cryptosporidium parvum | 10¹² |
| Bacillus cereus | 10⁸ |
| Campylobacter | 10⁹ |

The test results show that the pulse sterilisation apparatus according to the present invention operates in a very effective way.

## Claims

1. Pulse sterilisation apparatus (1) for sterilising a fluid product comprising a housing provided with an inlet piece (19) and an outlet piece (9), an inner electrode (2) positioned in between the inlet piece (19) and the outlet piece (9) and an outer electrode (31) positioned around the inner electrode (2), in which the space between the inner electrode (2) and outer electrode (31) forms a treatment chamber (21) for the fluid product, **characterised in that**,
the outer electrode (31) comprises a central bore of which the axis is parallel to a longitudinal direction of the pulse sterilisation apparatus (1) and in which the inner electrode (2) may be positioned,
in which the central bore has a conical shape and the inner electrode (2) comprises a middle section (24) which has a conical shape at least in the region of the outer electrode (31), and
in which the inner electrode (2) is adjustable in relation to the outer electrode (31).

2. Pulse sterilisation apparatus according to claim 1, **characterised in that** the conical shape of the central bore of the outer electrode (31) is equal to the conical shape of the middle section (24) of the inner electrode (2).

3. Pulse sterilisation apparatus according to claim 1 or 2, **characterised in that** the conical shape of the central bore of the outer electrode (31) and the conical shape of the middle part (24) of the inner electrode (2) are tapered from the side of the outlet piece (9) to the side of the inlet piece (19).

4. Pulse sterilisation apparatus according to claim 1, 2 or 3, **characterised in that** the outer electrode (31) is formed by a combination of at least one electrode plate (16) which is surrounded on both sides by isolation members (12) for electrically isolating the outer electrode (31) from the housing.

5. Pulse sterilisation apparatus according to one or the proceeding claims, **characterised in that** the housing and the inner electrode (2) are electrically connected to at least one connection lead (14, 15) for grounding the housing and the inner electrode (2).

6. Pulse sterilisation apparatus according to one or the proceeding claims, **characterised in that** the central bore of the outer electrode (31) and the inner electrode (2) are provided with a layer of dielectric material, such as ceramic material or plastic material, such as poly-ethylene or poly-propylene.

7. Pulse sterilisation apparatus according to one of the claims 4 through 7, **characterised in that** the pulse sterilisation apparatus (1) further comprises at least one high voltage supply (27, 29) for providing high voltage pulses to the at least one electrode plate (16) of the outer electrode (31).

8. Pulse sterilisation apparatus according to claim 7, **characterised in that** the at least one high voltage supply (27, 29) is driven by a pulse element (26).

9. Pulse sterilisation apparatus according to claim 7 or 8, **characterised in that** the at least one high voltage supply (27, 29) comprises a circuit amplifier (27) and a high voltage transformer (29) connected to the circuit amplifier.

10. Pulse sterilisation apparatus according to claim 9, **characterised in that** the high voltage transformer (29) is an ignition coil.

11. Pulse sterilisation apparatus according to claim 7 or 8, **characterised in that** the at least one high voltage supply (27, 29) comprises a thyristor circuit.

12. Pulse sterilisation apparatus according to one of the claims 7 through 11, **characterised in that** the at least one high voltage supply (27, 29) provides high voltage pulses with a pulse frequency of 50-1500 pulses/sec.

13. Pulse sterilisation apparatus according to one of the claims 7 through 12, **characterised in that** the at least one high voltage supply (27, 29) provides a voltage such that in the fluid product in the treatment chamber (21) an electrical field is created of between 10 and 50 kV/cm.
